(19)

**Europäisches Patentamt**

**European Patent Office**

**Office européen des brevets**

(11) **EP 4 725 578 A1**

(12) # EUROPEAN PATENT APPLICATION

(43) Date of publication:
**15.04.2026 Bulletin 2026/16**

(21) Application number: **25206964.6**

(22) Date of filing: **06.10.2025**

(51) International Patent Classification (IPC):
**B01D 15/10** *(2006.01)*    **B01D 15/18** *(2006.01)*
**B01D 15/22** *(2006.01)*    **B01D 15/38** *(2006.01)*
**C07K 1/22** *(2006.01)*    **B01L 3/00** *(2006.01)*
**G01N 30/60** *(2006.01)*

(52) Cooperative Patent Classification (CPC):
**B01D 15/18; B01D 15/22; B01D 15/3804;**
**C07K 1/22**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **08.10.2024 EP 24205275**
         **08.10.2024 EP 24205282**

(71) Applicant: **TQ Therapeutics GmbH**
**81245 Munich (DE)**

(72) Inventor: **RADISH, Sabine**
**83075 Bad Feilnbach (DE)**

(74) Representative: **Schiweck Weinzierl Koch**
**Patentanwälte Partnerschaft mbB**
**Ganghoferstraße 68 B**
**80339 München (DE)**

(54) **COLUMN DESIGNS FOR AFFINITY CHROMATOGRAPHY**

(57) This invention relates to a column (1) for binding a cell, the column (1) comprising a column body comprising a retention volume comprising a stationary phase, wherein preferably the stationary phase is an affinity chromatography matrix; at least one open end for adding or removing the cell to or from the column (1); and at least one obstacle within the column body, wherein the at least one obstacle is a grid or a pin; optionally wherein the column has a first open end and a second open end arranged opposite to each other on the column. The present invention also relates to a system of columns (1). The present invention also relates to a method for binding a cell.

EP 4 725 578 A1

**Description**

**CROSS-REFERENCE TO RELATED APPLICATION**

The present application claims the benefit of priority of EP Patent Application No. 24 205 275.1 filed 8 October 2024 and of EP Patent Application No. 24 205 282.7 filed 8 October 2024, the content of which is hereby incorporated by reference in its entirety for all purposes.

**FIELD OF THE INVENTION**

This invention relates to a column for binding a cell, the column comprising a column body comprising a retention volume, at least one open end for adding or removing the biological entity to or from the column, and at least one obstacle within the column body, wherein the at least one obstacle is a grid or a pin. The present invention also relates to a method for binding a cell. Furthermore, the invention relates to a system of columns.

**BACKGROUND**

Cellular therapy offers numerous advantages in the treatment of various diseases, including cancer, autoimmune diseases, and infectious diseases. By genetically modifying a patient's T cells to express chimeric antigen receptors (CARs), these cells are engineered to specifically target and destroy desired cells in the patient's body, leading to highly personalized and effective treatment options. One of the most significant benefits of this therapy is the capability to achieve complete remission in patients with certain types of blood cancers, such as acute lymphoblastic leukaemia and diffuse large B cell lymphoma, where conventional treatments have failed.

Current manufacturing strategies for cellular therapy products, such as CAR T cells or T cells expressing recombinant T cell receptors (TCR), are often time-consuming and costly. The complexity of the current manufacturing process, which involves multiple handling steps and extensive *in vitro* cultivation, manipulation, and expansion, in addition to rigorous product testing before the product can be administered put significant burden on the accessibility of the product. Moreover, current cellular therapy products require centralized manufacturing at specialized facilities, which involves significant logistical challenges. These factors contribute to high costs and delays in manufacturing, raising concerns about the availability and affordability of these therapies for both healthcare systems and patients, especially late-stage patients, where every day counts.

Thus, these life-saving therapies are currently not easily scalable and as a result are limited to a fraction of patients in need of such treatment.

One promising strategy to reduce time and cost is the "allogeneic approach", which uses novel gene editing methods to create universal donor T cells, engineered to avoid rejection by the patient's immune system and thus prevent graft-versus-host reactions (Perez et al., 2020). However, this approach requires extensive genetic modification to circumvent immune responses, and it carries a significant risk of clinical toxicity. Following a fatality in the trial with UCARTCS1A by Cellectis in 2021, the FDA halted all clinical trials using universal donor cells due to safety concerns (Locke et al., 2021).

An alternative strategy is the *in vivo* generation of manipulated cells, such as T cells, through systemic or localized application of viral or non-viral gene delivery systems carrying the relevant genes. These delivery systems or nanocarriers can be produced using standardized methods, potentially broadening their clinical application compared to conventional cellular therapies. While recent studies have shown some anti-tumoral responses in humanized mouse models, they also revealed method-associated side effects, including high immunogenicity of the delivery systems and their cargo, rapid hepatic and renal clearance, pre-existing anti-viral vector immunity, limited distribution within the body, and unspecific targeting of non-T cells, which significantly impacts the treatment's safety profile. See, for example, Xin et al., 2022.

Therefore, optimized manufacturing of cellular products, such as CAR T cells, remains a hot topic. International patent applications WO 2019/217964 A1 and WO 2022/072885 A1 describe a closed-loop system for modifying patient cells. In this system, a cell-containing sample, such as a blood sample, is processed through a cell separation module, where cells can be enriched. The separated cells are then modified using therapeutic agents and reinfused into the patient. These documents also describe the activation of CAR T cells, for example, through CD3 and CD28 costimulation, before administration.

Additionally, WO 2023/122277 A2 discloses a rapid method for generating CAR T cells by simultaneously activating T cells in a mononuclear cell sample and exposing them to a viral vector for transduction with a CAR construct. Despite these advancements, the current cellular therapy products face significant barriers and there remains a need for improved methods that allow for the easy manufacturing of patientderived cellular therapy products, such as genetically modified CAR T cells.

The document US 2018/178142 A1 discloses cell chromatography columns, which serve for isolating target cells and using cells as a stationary phase for analyte reagents which interact with cells in the stationary phase. The columns comprise a bed of medium onto which the cells are captured, the bed is retained in the column by two frits, one below and the other one above the bed. Obstacles like pins are avoided, because they could damage cells to be isolated. A similar disclosure can be found in WO 2016/100447 A1, which discloses a device and a method for cleaning samples prior to mass spectrometry analysis.

Therefore, there is a need in the art for methods and devices for cell therapy manufacture, that would allow generation of CAR T cell therapy in a cost and time efficient manner.

## SUMMARY OF THE INVENTION

The present invention therefore addresses this need and provides a solution as described herein, shown in the examples, illustrated in the figures and as defined in the claims.

In a first aspect, the present invention relates to a column for binding a cell, the column comprising a column body comprising a retention volume comprising a stationary phase, wherein preferably the stationary phase is an affinity chromatography matrix, at least one open end for adding or removing the cell to or from the column, and at least one obstacle within the column body, wherein the at least one obstacle is a grid or a pin, optionally wherein the column has a first open end and a second open end arranged opposite to each other on the column.

In one embodiment of the first aspect, the binding of the cell is for the enrichment of the cells in a sample or for depletion of cells a sample.

In yet another embodiment of the first aspect, the column is part of a tubing set, preferably wherein the column is part of a target cell retention reservoir, a genetic modification unit, and/or a target cell washing and formulation unit of the tubing set.

In a second aspect, the present invention relates to a system for binding of a cell from a sample comprising two or more columns arranged in a line and/or in parallel, the system further comprises one or more pumps, a sample filling unit, and optionally a washing unit, wherein at least one column is a column of the first aspect.

In a third aspect, the present invention provides the column of the first aspect or the system of the second aspect for use in *in-vitro* diagnosis or in a method of producing a cellular therapy product.

In a fourth aspect, the present invention relates to a method of producing a cellular therapy product comprising the use of the column of the first aspect or the system of the second aspect.

In a fifth aspect the invention provides a method for binding a cell comprising providing at least one column of the first aspect, adding a sample comprising the cell through the at least one open end of the column, and removing the enriched cells from the sample, optionally wherein the at least one column is part of the system of the second aspect.

In one embodiment of the fifth aspect, the sample comprises whole blood or peripheral blood mononuclear cells.

In another embodiment of the fifth aspect, the method of the invention is used in a method of producing cellular therapy product.

In yet another embodiment of the fifth aspect, the method of the invention is used for *in-vitro* diagnostic or therapeutical purposes.

## BRIEF DESCRIPTION OF THE DRAWINGS

**Fig. 1: Examples of columns for binding cells with a bicone-cylinder shape and obstacles arranged within.** A to E, different designs of the column of the invention. Dotted lines represent a mesh (e.g. Nylon mesh), dashed lines represent a grid, and solid lines represent pins.

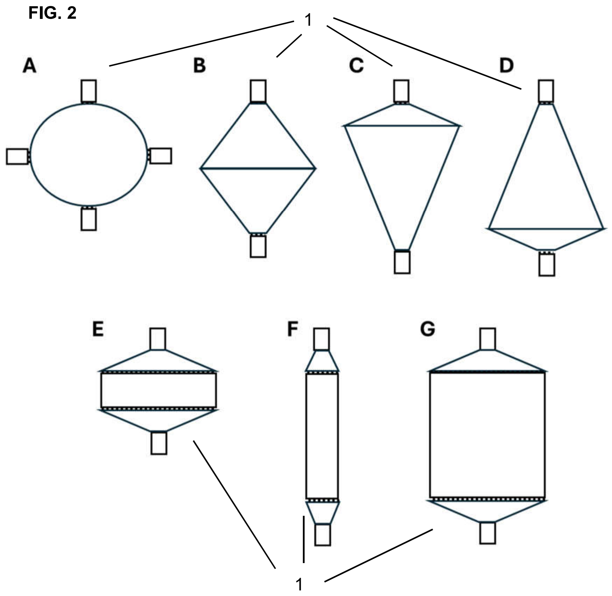

**Fig. 2: Examples of columns for binding cells with spherical, biconical, and bicone-cylinder shapes.** A to G, different designs of the column. Dotted lines represent a mesh (e.g. Nylon), dashed lines represent a grid, and solid lines represent pins.

**Fig. 3: Results of CD3+ cells enrichment using the column of the invention.** A, CD3+ cell purity; B, CD3+ cell count. Four independent runs are shown.

**Fig. 4: Results of CD4+ T cell enrichment from PBMC material with different column geometries.** The behaviour of the different column geometries was evaluated by comparing their CD4+ cell selection performance out of peripheral blood mononuclear cells (PBMCs) using a peristaltic pump and keeping the volumetric flow rate of the peristaltic pump constant. A, column 1 to 5 with different column geometries; B, cell counts of positive fractions.

**Fig. 5: Laminar flow through** a **column with no obstacles.** A coloured liquid was flushed through a column without any obstacles with a pump at a speed of 30 ml/min. A, schematic of the column. B-H, laminar flow through the column. Images were taken at 2 second intervals.

**Fig. 6: Non-laminar flow through a column with two grids.** A coloured liquid was flushed through the column containing two grids (an upper grid and a lower grid) with a pump at a speed of 30 ml/min. A, detail of a 3D printed grid. B-L, non-laminar flow through the column. Images were taken at 2 second intervals.

**Fig. 7: Non-laminar flow through a column with four pins.** A coloured liquid was flushed through the column containing four pins with a pump at a speed of 30 ml/min. A-C, detail of the 3D printed pins. D-N, non-laminar flow through the column. Images were taken at 2 second intervals.

**Fig. 8: Series arrangements of columns of the system of the present invention.** A, an example serial configuration of four columns and a single pump; B, an example series configuration of three columns, one wash unit, and one pump; C, an example series configuration of two columns, one wash unit, and two pumps. 1, column; 2, pump; 3, wash unit.

**Fig. 9: Parallel arrangements of columns of the system of the present invention.** A, an example parallel configuration of four columns operated by four pumps; B, an example parallel configuration of four columns operated by one pump. 1, column; 2, pump; 3, wash unit.

**Fig. 10: Series-parallel arrangements of columns of the system of the present invention.** A, an example series-parallel configuration of four columns operated by two pumps; B, an example series-parallel configuration of four columns, one wash unit and two pumps. 1, column; 2, pump; 3, wash unit.

## DETAILED DESCRIPTION

There is a need in the art for improved methods and devices to allow faster and more efficient selection of target cells.

Current affinity chromatography columns do not offer the optimal binding conditions necessary for fast and efficient selection of target cells from whole blood. Specifically, current chromatography columns feature laminar flow of the sample through the column, resulting in an inefficient selection of the cells on the affinity chromatography column as well as sedimentation of the cells and forming a packed matrix bed in the column. Therefore, target cell retention reservoirs and affinity chromatography columns are one of the limiting factors that hinder upscaling of the cell therapy technology, such as CAR T cells, and thus limit patients from accessing life-saving medication.

The present invention solves this problem and provides a column with improved characteristics to achieve the need for optimised, e.g. faster and more efficient, selection of cells. The columns of the present invention feature designs with an optimized non-laminar flow of the sample inside the column for better selection of target cells, and for better elution of the target cells from the column.

The inventors of the present invention have surprisingly found that adding obstacles into the affinity chromatography column achieves sample flow with non-laminar characteristics within the column body. Surprisingly, such optimized non-laminar flow results in better binding of cells to the resin within the column. Therefore, the column of the present invention is particularly suited for fast and efficient selection of cells from a biological material. The column of the invention may *inter alia* be used in a bedside CAR T cell generation system, where it speeds up the selection of T cells from peripheral blood of the patient and thus facilitates the process of manufacturing tailored cell therapy.

Accordingly, the present invention relates in a first aspect to a column for binding a cell, the column comprising a column body comprising a retention volume comprising a stationary phase, wherein preferably the stationary phase is an affinity chromatography matrix, at least one open end for adding or removing the cell to or from the column, and at least one obstacle within the column body, wherein the at least one obstacle is a grid or a pin, optionally wherein the column has a first open end and a second open end arranged opposite to each other on the column.

Thus, a column of the first aspect comprises at least one obstacle positioned in the column body, wherein the at least one obstacle is a grid or a pin.

In some embodiments, the column of the first aspect comprises at least 2, at least 3, at least 4, at least 5, or at least 6 obstacles. Preferably, the column comprises at least 2, at least 3, or at least 4 pins, or at least 5 pins, at least 6 pins, at least 7 pins, at least 8 pins, at least 9 pins, at least 10 pins, at least 11 pins, at least 12 pins, at least 13 pins, at least 14 pins, or at least 15 pins.

As used herein, "pin" refers to an inward protrusion in the column body. The protrusion may be formed by the geometry of the column body itself (such as a "dent" in the column body) or may be formed by an object positioned in the column body. Preferably, the pin has the shape of a rectangle or a triangle (wedge) on a longitudinal cross-section of the column. In some embodiments, the pin is arranged at an angle between about 90° and about 100° to the longitudinal or transversal axis of the column. Thus, the pin may be positioned in the column body perpendicularly to the longitudinal axis of the column, or slightly inclining downwards. In embodiments of the column of the invention where the pin has a wedge shape on the longitudinal cross-section of the column, the wedge may be an isosceles triangle, or the tip of the triangle may slightly incline downwards. The triangle shape may have a tip angle of about 20° to about 60°, such as 25°, 30°, 35°, 45°, 50°, or 55°.

The pin has a length between about 25% and about 100% of the column diameter, has a width between about 25% and about 50% of the column diameter, and has a thickness between about 2 mm and about 8 mm. As used herein, length refers to the longest dimension of the obstacle, width refers to the second longest dimension of the obstacle, and thickness refers to the smallest dimension of the obstacle. Preferably, the pin has a length between about 33% and about 66% of the column diameter, and has a width between about 33% and about 50% of the column diameter. Preferably, the pin has a length equal to the column diameter, and has a width of about 50% of the column diameter. Preferably, the pin has a length between about 33% and about 50% of the column diameter, and width between about 33% and about 50% of the column diameter.

In some embodiments of the column of the first aspect comprises two or more pins, each subsequent pin is arranged in the opposite direction and with an offset to a preceding pin. Thus, the pins in the column body may be arranged in an alternating configuration, for example wherein the first pin is arranged on one side of the column body, and the second pin is arranged on the opposite side of the column body, and the third pin is arranged on the same side of the column body as the first pin, etc. The offset from one pin to another pin, such as the offset from the first pin located on one side of the column to the second pin located on the other side of the column, may be selected such that the pins are evenly spaced across a dimension of the column, wherein the dimension is preferably longitudinal or transversal axis of the column. As an illustrative example, the first pin may be located at a distance from one end of the column, the last pin may be located at the same distance from the other end of the column, and the distance from the fist pin to the last pin, or any pins in between, is the same distance as the distance from either end to a respective pin. The offset may also be arbitrarily selected such that the pins are not evenly spaced across the column.

In some embodiments, the column comprises at least 2, at least 3, at least 4, at least 5, or at least 6 grids, or at least 7 grids, at least 8 grids, at least 9 grids, or at least 10 grids.

As used herein, "grid" refers to a structure comprising a surface and a number of perforations in the surface. Preferably, the surface is arranged at an angle between about 90° and about 100° to the longitudinal, transversal, and/or diagonal section of the column, wherein the surface extends across the column or a portion thereof, the surface comprising two or more perforations, and wherein the total area of the perforations is between about 25% and about 75% of the area of the surface, and wherein the grid has a thickness of about 1 mm to about 8 mm.

Preferably, the grids are arranged parallel to each other. Preferably, the at least one grid is arranged perpendicular to the column's longitudinal axis.

Preferably, the grid is arranged at an angle between about 90° and about 100° to the longitudinal axis of the column, such as about 91°, about 92°, about 93°, about 94°, about 95°, about 96°, about 97°, about 98°, or about 99°. Preferably, the grid is arranged at an angle between about 90° and about 100° to the transversal axis of the column, such as about 91°, about 92°, about 93°, about 94°, about 95°, about 96°, about 97°, about 98°, or about 99°. Preferably, the grid is arranged at an angle between about 90° and about 100° to the diagonal axis of the column, such as about 91°, about 92°, about 93°, about 94°, about 95°, about 96°, about 97°, about 98°, or about 99°.

Preferably, the grid comprises a perforated surface, wherein the total area of perforations is about 30%, about 35%, about 40%, about 45%, about 50%, about 55%, about 60%, about 65%, or about 70%. The perforations in the grid may be round, rectangular, or square. As an illustrative example, the diameter of a round perforation may be between about 5% and about 30% of the column diameter, such as about 8%, about 10%, about 12%, about 14%, about 16%, about 18%, about 20%, about 22%, about 24%, about 26%, or about 28%. Preferably, the diameter of the perforation is about 14% of the column diameter.

Preferably, the grid extends across the column, such as across the width of the column. Preferably, the grid extends across a portion of the column, such as across a part of the column, such as a portion defined by a particular column geometry defined herein. For example, the grid may extend across a portion of the diagonal axis of the column, such as diagonally across the cone portion of the column having a shape of a bicone cylinder.

The present disclosure envisages that the obstacles positioned within the column body achieve a non-laminar flow through the column. Preferably, the non-laminar flow of the cells in the column body results in optimized binding of the cells compared to laminar flow thereof.

Non-laminar flow within the column is achieved by the placement of obstacles within the column body that disrupt the otherwise streamlined, laminar movement of fluid through the retention volume. These obstacles, which may take the form of pins or grids, create localized changes in fluid velocity and direction, leading to vortices, eddies, and turbulent mixing zones. As the cell passes through these disturbed flow regions, i.e. regions with a turbulent flow or a non-laminar fluid dynamic, its trajectory is repeatedly altered, preventing uniform channelling or sedimentation that typically occurs under conditions of a laminar flow of the fluid with the cells. The resulting chaotic and heterogeneous flow pattern increases the probability of contact between the cells and the binding matrix, thereby enhancing binding efficiency, improving overall yield, and ensuring a more homogeneous distribution across the column.

Thus, the column of the first aspect has the advantageous effect that the flow of the cell, e.g. blood cells in a suitable medium, through the column occurs in a non-laminar and turbulent manner. Preferably, the optimized binding of the cells is the enrichment of the cells in a sample and a higher cell purity and/or higher cell yield. Therefore, such non-laminar flow has the benefit, *inter alia,* of faster and more efficient selection of the cells. Furthermore, one of the benefits of the non-laminar flow is lower sedimentation and homogeneous selection of the cells throughout the affinity chromatography matrix. Furthermore, the non-laminar flow could be one-directional or bi-directional within the column body.

Preferably, the cell is a blood cell, preferably a CD3+ blood cell. CD3 (cluster of differentiation 3) is a protein complex and T cell co-receptor that is involved in activating both the cytotoxic T cell (CD8+ naive T cells) and T helper cells (CD4+ naive T cells). More preferably, the cell is a CD4+ blood cell and/or CD8+ blood cell.

Preferably, the cell is an antigen expressing cell in a suitable buffer. An antigen expressing cell in a suitable buffer may be any cell in an appropriate medium for that cell, for example an *in vitro* cultured T cell, B cell, or NK cell in a suitable buffer.

The retention volume of the column body comprises a stationary phase, preferably an affinity chromatography matrix.

As used herein, the term "stationary phase" refers to the part of a chromatographic system through which the mobile phase flows and where distribution of the components contained in the liquid phase (either dissolved or dispersed) between the phases occurs. The term "affinity chromatography matrix" refers to stationary phase which has an affinity reagent immobilized thereon.

Preferably, the column body has the shape substantially of a sphere, capsule, ellipsoid, bicone, conical cylinder, biconical cylinder, cylinder, or a polyhedron. As used herein, the term "sphere" refers to a perfectly round geometrical object in three-dimensional space wherein all points on the surface are equidistant from the centre, with volume $V = \frac{4}{3}\pi r^3$ and surface area $A = 4\pi r^2$. As used herein, the term "capsule" refers to a three-dimensional shape consisting of a cylinder with hemispherical ends with volume $V = \pi r^2 h + \frac{4}{3}\pi r^3$, where h is the height of the cylindrical part, and surface area $A = 2\pi rh + 4\pi r^2$. As used herein, the term "ellipsoid" refers to a three-dimensional shape similar to a sphere but stretched along one or more of its axes, with volume $V = \frac{4}{3}\pi abc$, where a, *b,* and c are the semi-axes lengths, and surface area $A \sim 4\pi \left(\frac{a^p b^p + a^p c^p + b^p c^p}{3}\right)^{\frac{1}{p}}$, where $p \sim 1.6075$. As used herein, the term "bicone" refers to a shape formed by joining two identical cones at their bases, with volume $V = \frac{2}{3}\pi r^2 h$, where h is the height of the cone. As used herein, the term "conical cylinder" refers to a shape with two circular ends of different diameters, also referred to as frustrum of a cone, with volume $V = \frac{1}{3}\pi h(r_1^2 + r_1 r_2 + r_2^2)$, where $r_1$ and $r_2$ are the radii of the two bases, and *h* is the height, and with surface area $A = \pi(r_1 + r_2)\sqrt{(r_2 - r_1)^2 + h^2} + \pi r_1^2 + \pi r_2^2$. As used herein, the term "biconical cylinder" refers to a shape combining a central cylindrical section with conical ends, with volume $V = \pi r^2 h_c + \frac{2}{3}\pi r^2 h_k$, where $h_c$ is the height of the cylindrical section and $h_k$ is the height of each cone. As used herein, the term "cylinder" refers to a shape with straight parallel sides and circular ends of equal diameter with volume $V = \pi r^2 h$ and surface area $A = 2\pi(r + h)$. As used herein, the term "polyhedron" refers to a three-dimensional shape with a number of flat polygonal faces, straight edges, and sharp vertices. Examples of the polyhedron shape is a tetrahedron, a cube, octahedron, dodecahedron, icosahedron.

Preferably, the bicone, conical cylinder or biconical cylinder comprise truncated cones. Preferably, the bicone or biconical cylinder comprise two cones with different dimensions. Preferably, the column body has the shape substantially of the cylinder and a ratio of a column body diameter to column body length is lower than 0.1, or is between 0.1 and 0.01. Preferably, the column body has the shape substantially of cylinder and a ratio of a column body diameter to column body length is higher than 0.1, or is between 1 and 0.1.

Preferably, the at least one obstacle within the column body is a grid or a pin. The obstacles may be positioned inside the column to prevent sedimentation and formation of a packed matrix bed, thus allowing optimal distribution of the biological material in the affinity chromatography matrix as well as optimized flow dynamics (both one- and bi-directional) of blood and cells containing buffers for optimized cell purity and cell yield. Additionally, one or more grids may be positioned inside the column to prevent the formation of a packed matrix bed and allowing better movement and mixing of the matrix and optimized flow dynamics for better cell purity and cell yield. Furthermore, one or more meshes may be positioned inside the column. For example, one or more meshes are positioned at each end of the column to allow better flow dynamics (one- and bi-directional) for the medium, e.g. blood, when entering the column through the open end.

Preferably, the at least one obstacle, i.e. a pin or grid, is positioned within the column body substantially perpendicularly, diagonally or horizontally to the longitudinal axis of the column body, as described above. Preferably, the at least one obstacle extends at least across a portion of the column body or across the entire column body substantially perpendicularly, diagonally or horizontally to the longitudinal axis of the column body. Preferably, the mesh is positioned perpendicularly between the at least one open end and the column body. Preferably, the mesh is positioned perpendicularly in the column body to the longitudinal axis of the column body.

Preferably the mesh is a web, a filter instead of a web is also possible but not preferred. The mesh has a mesh size or a pore size which will hold the stationary phase in the column body but let the biological entity pass through it. Preferable mesh sizes or pore sizes are between about 5 $\mu$m and about 50 $\mu$m. Further applicable mesh sizes are between about 5 $\mu$m and about 40 $\mu$m, or between about 5 $\mu$m and about 30 $\mu$m, or between 10 $\mu$m and 40 $\mu$m. In general. The material of the mesh must be sterilisable, because the columns equipped with the mesh may be used in biological or medical procedures using cells. Preferably the mesh could be sterilised using gamma rays or x-rays or e-beam. The material of the mesh preferably withstands temperatures above 50 °C without any damage or impact on the function of the mesh. Useful materials of the mesh may be polyamide (nylon), polyester, polypropylene, perfluoroalkoxy alkane polymers (PFA), polyether ether ketone (PEEK), fluorinated ethylene propylene (FEP), or other suitable materials.

Preferably, the column has a first open end and a second open end arranged opposite to each other on the column. Therefore, the first open end may serve as an inlet of the column through which the cell enters the column, and the second open end may serve as an outlet of the column, through which the medium exits the column after the cell has been enriched on the affinity matrix. However, depending on the application of the column, the flow through the column may change periodically, such that the second open end becomes an inlet of the column, and the first open end becomes the outlet of the column. In this configuration, the at least one mesh is preferably positioned between the first open end and the column body and between the second open end and the column body, wherein the mesh is oriented perpendicularly to the longitudinal axis of the column body. As described elsewhere herein, this serves to achieve the non-laminar flow throughout the column.

Preferably, the binding of the cell is for the enrichment of the cell in a sample or for depletion of the cell from a sample. Accordingly, the column of the invention may be used to select a target cell population from a sample, the target cell population is then enriched in the eluate after having been eluted from the matrix. An illustrative example may be CD3+ cells extraction from a blood sample, which, after elution from the column, are then used for CAR T cell generation. Furthermore, the column may be used to deplete or remove a target cell population from the sample. An illustrative example of such application may be depletion of CD20+ cells from blood of a patient suffering from blood cancer, such as B cell lymphoma or leukaemia. The blood free from cancerous CD20+ cells is then returned to the patient and isolated CD20+ cells are discarded.

The present invention relates in a second aspect to a system for binding of a cell from a sample comprising two or more columns arranged in a line and/or in parallel, the system further comprises one or more pumps, a sample filling unit, and optionally a washing unit, wherein preferably at least one column is a column of the first aspect.

The complete disclosure of the invention of the first aspect is herein incorporated by reference.

Thus, disclosed herein is a system of multiple columns for binding of a cell from a sample. The system comprises two or more columns, each column having a first open end functions as an inlet of the column and a second open end functions as an outlet of the column and a column body, wherein the column body comprises a retention volume. The columns are arranged in a line and/or in parallel within the system of columns. The system further comprises one or more pumps, a sample filling unit, and optionally a washing unit. Preferably, the at least one column comprised in the system of the second aspect is a column of the first aspect according to any one of the embodiments of the first aspect described herein. The cell has one or more epitopes suitable for binding to an affinity chromatography matrix.

As used herein, the term "epitope", also known as the "antigenic determinant", refers to a portion of an antigen to which an antibody or any other molecule specifically binds, thereby forming a complex. Thus, the term "epitope" includes any molecule or protein determinant capable of specific binding to an immunoglobulin, T-cell receptor, or to another molecule, which is not an immunoglobulin. The binding site(s) (paratope) of an antibody molecule may specifically bind to/interact with conformational or continuous epitopes, which are unique for the target structure. Epitopic determinants usually consist of chemically active surface groupings of molecules such as amino acids or sugar side chains and usually have specific three-dimensional structural characteristics, as well as specific charge characteristics. Epitope determinants may include chemically active surface groupings of molecules such as amino acids, sugar side chains, phosphoryl, or sulfonyl, and, in certain embodiments, may have specific three-dimensional structural characteristics, and/or specific charge characteristics. With regard to polypeptide antigens a conformational or discontinuous epitope is characterized by the presence of two or more discrete amino acid residues, separated in the primary sequence, but assembling to a consistent structure on the surface of the molecule when the polypeptide folds into the native protein/antigen. The two or more discrete amino acid residues contributing to the epitope may be present on separate sections of one or more polypeptide chain(s). These residues come together on the surface of the molecule when the polypeptide chain(s) fold(s) into a three-dimensional structure to constitute the epitope. In contrast, a continuous or linear epitope comprises two or more discrete amino acid residues, which are present in a single linear segment of a polypeptide chain. As an illustrative example, a "context-dependent" CD3 epitope refers to the conformation of said epitope. Such a context-dependent epitope, localized on the epsilon chain of CD3, can only develop its correct conformation if it is embedded within the rest of the epsilon chain and held in the right position by heterodimerization of the epsilon chain with either CD3 gamma or delta chain. In contrast thereto, a context-independent CD3 epitope may be an N-terminal 1-27 amino acid residue polypeptide or a functional fragment thereof of CD3 epsilon. Generally, epitopes can be linear in nature or can be a discontinuous epitope. Preferred examples of an epitope comprise surface molecules naturally expressed on target cells, such as CD markers (CD3, CD9), or an affinity tag (or epitope tag) expressed on target cells, for example by expressing a fusion protein between a naturally expressed molecule on the target cell and the affinity tag. Examples of suitable epitope tags include, but are not limited to, Alfa-tag, FLAG-tag, His6-tag, Strep-tag, Myc-tag, HA-tag, VSV-G-tag, HSV-tag, V5-tag, SPOT-tag, BC2 tag, and EPEA tag. The antigen may also be a protein, for example, glutathione-S-transferase (GST), maltose binding protein (MBP), chitin binding protein (CBP) or thioredoxin.

As used herein, "sample filling unit" refers to a dedicated unit for loading the sample to the system. Such sample filing unit may be for example a connector matching the end of a syringe, blood bag, a bottle etc. In a linear arrangement of columns, the sample filling unit has preferably at least one fluid connection to the inlet of the first column of the line of columns for sample application, or the sample filling unit may be the inlet of the first column in the line of columns. In a parallel arrangement of the columns the sample filling unit is fluidly connected with the first open ends of the columns of the parallel arrangement.

As used herein, "washing unit" refers to a compartment serving to wash the previously bound and eluted cell(s). The washing unit may be a column as described elsewhere herein without a stationary phase. The washing unit may also be any other technical unit.

The system of columns of the second aspect may further comprise a sample formulation unit and/or a sample reinfusion unit. The "sample formulation unit" refers to a compartment in which a sample could be formulated for further processing, including preparation of an injectable solution, e.g. by buffer exchange. The "sample reinfusion unit" refers to a compartment with which an injectable solution of a cellular therapy product could be reinfused in a patient.

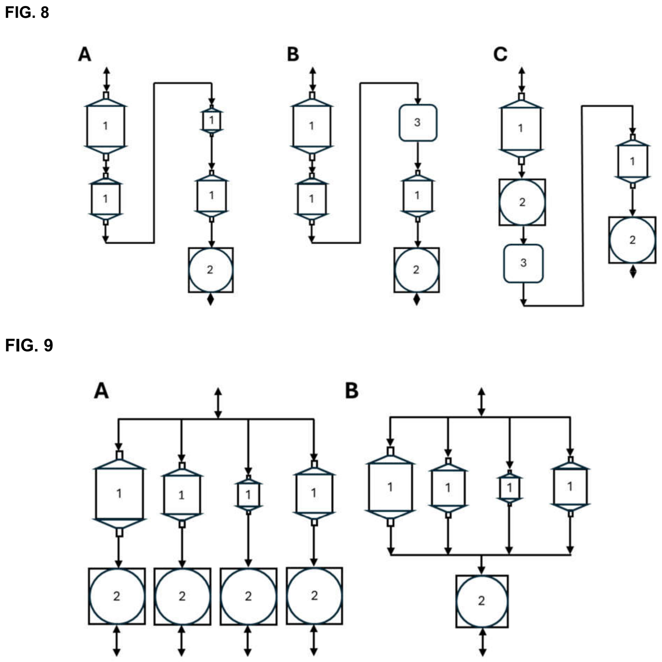

The columns of the system may be arranged in a line in that the outlet of one column is fluidly connected with the inlet of another column. The outlet of the one column could also be fluidly connected with an inlet of a pump and an outlet of the pump is fluidly connected with the inlet of the other column. It may also be possible that the outlet of the pump is not connected with the inlet of the other column. The outlet of the other column or the outlet of the pump may also be connected with a sample collecting unit. Thus, any number of columns may be arranged in a serial configuration or arrangement, as exemplary shown in Figure 8.

Preferably, the pump is arranged after every column or after every 2nd, 3rd, 4th, 5th, 6th or Nth column of the system, wherein the outlet of the last pump of the line of columns of the system is or is not connected with the inlet of the further column and wherein N is preferably any natural number between 2 and 10. The number of columns, N, could in general be any natural number that makes technical sense in order to achieve the desired goal of using the system of columns of the second aspect. N may be 2, 3, 4, 5, 6, 7, 8, 9, 10, 11, 12, 13, 14, 15 or more. Thus, N could be any natural number between 2 and 50, between 2 and 45, between 2 and 40, between 2 and 35, between 2 and 30, between 2 and 20 and preferably between 2 and 10. Thus, in a system comprising a serial configuration of columns, a number of pumps may be arranged in the series of columns. A particular advantage of arranging a pump after a section of the system, i.e. a section comprising a number of columns, is the possibility to operate the flow through that section of the system independently of the other sections of the system.

Preferably, the retention volume of the at least one column, as part of the linear or parallel arrangement of columns within the system of columns of the second aspect, comprises a stationary phase, preferably an affinity chromatography matrix for binding at least one epitope of the cell(s), and wherein the stationary phases of the columns have the same or different binding affinities. As used herein, the term "stationary phase" refers to the part of a chromatographic system through which the mobile phase flows and where distribution of the components contained in the liquid phase (either dissolved or dispersed) between the phases occurs. The term "affinity chromatography matrix" refers to stationary phase which has an affinity reagent immobilized thereon.

The system of multiple columns of the second aspect further comprises the parallel arrangement of the columns. To use the columns in parallel their first open ends are fluidly connected with the sample filling unit. The features of the columns may be the same as described above for the linear arrangement of columns. Thus, also the columns of the parallel arrangement of columns may comprise obstacles and/or the stationary phase as described above for the linear arrangement of columns.

Preferably, a group of columns of the parallel arrangement of columns is fluidly connected via their outlets with exactly one pump. This is exemplary shown in Figure 9B. Thus, the flow through the group of columns is constant for each column in the group of columns and is operated simultaneously via the one pump.

Preferably, a group of columns of the parallel arrangement of columns is fluidly connected via their outlets with a group of pumps in that exactly one column is assigned to one pump. Thus, the flow through each column in the system may be operated independently. This is exemplary shown in Figure 9A. A particular advantage of this configuration is the flexibility of the system, wherein the flow through a column can be increased, decreased, or reversed. A further advantage is that different cell types could be selected with each column, which may depend on the use of different affinity chromatographic matrices.

Preferably, the system of parallel arranged columns comprises a group of columns with a single pump or a group of columns with a number of pumps equal to the number of columns in the group of columns. Preferably, the system comprises a combination of both groups of columns.

Preferably, the system of columns, independent of their arrangement, comprises 2, 3, 4, 5, 6, 7 or N columns, wherein N is any natural number preferably between 2 and 10. The number of columns, N, could in general be any natural number that makes technical sense in order to achieve the desired goal of using the system of columns of the present invention. N may be 2, 3, 4, 5, 6, 7, 8, 9, 10, 11, 12, 13, 14, 15 or more. Thus, N could be any natural number between 2 and 50, between 2 and 45, between 2 and 40, between 2 and 35, between 2 and 30, between 2 and 20 and preferably between 2 and 10.

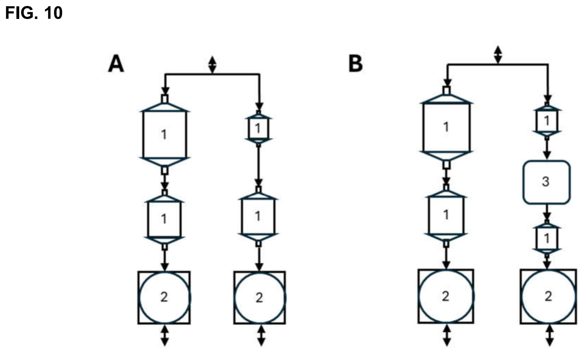

The system of columns may also comprise a combination of linear and parallel arrangements of columns. Therefore, the system may comprise at least two lines of columns, wherein the columns of each line are arranged as described above for linear column arrangements and having at least one pump in each line and wherein the at least two lines of columns are arranged in parallel. One line of columns of this system equals one column in the parallel arrangement described before and each inlet of the first column of each line of columns is fluidly connected to the sample filling unit. Such a system is exemplary shown in Figure 10. In this system, preferably the number of lines of columns is between 2 and 10, or between 2 and 9, or between 2 and 8, or between 2 and 7, or between 2 and 6, or between 2 and 5, or between 2 and 4, or between 2 and 3, or wherein the number of lines of columns is 2. The number of columns within one line may vary and may comprise 2, 3, 4, 5, 6, 7 or N columns, wherein N is any natural number preferably between 2 and 10 as described before.

Current chromatography columns feature laminar flow of the sample through the column, resulting in an inefficient selection of the biological entity on the affinity chromatography column as well as sedimentation of the biological material and forming a packed matrix bed in the column.

The present invention solves this problem and provides a system of columns with improved characteristics to achieve the need for optimized, e.g. faster and more efficient, selection of cells. The columns of the system of the present invention preferably feature designs with an optimized non-laminar flow of the sample inside the column for better selection of target cells, and for better elution of the target cells from the column. This is further described in the first aspect, which disclosure is incorporated herein by reference.

Adding obstacles into the affinity chromatography column achieves sample flow with non-laminar characteristics within the column body. Such optimized non-laminar flow results in better binding of cells to the resin within the column. Such columns, especially columns of the first aspect, are therefore preferably used in the system of columns of the present invention.

Preferably, the flow through of the at least one column is uni-directional or bi-directional.

Preferably, the column body of the at least one column comprises at least one obstacle, wherein the at least one obstacle is arranged to achieve a non-laminar flow of the biological entity in the column body.

Preferably, the at least one obstacle within the column body is a grid or a pin, as e.g. further defined in the first aspect. The pins may be preferably positioned inside the column to prevent sedimentation and formation of a packed matrix bed, thus allowing optimal distribution of the cells in the affinity chromatography matrix as well as optimized flow dynamics (both one- and bi-directional) of blood and cells containing buffers for optimized cell purity and cell yield. Additionally, one or more grids may be positioned inside the column to prevent the formation of a packed matrix bed and allowing better movement and mixing of the matrix and optimized flow dynamics for better cell purity and cell yield. Furthermore, one or more meshes are positioned inside the column. For example, one or more meshes are positioned at each end of the column.

Preferably, the at least one obstacle, i.e. a pin or a grid, is positioned within the column body substantially perpendicularly, diagonally or horizontally to the longitudinal axis of the column body. Preferably, the at least one obstacle extends at least across a portion of the column body or across the entire column body substantially perpendicularly, diagonally or horizontally to the longitudinal axis of the column body. Preferably, the mesh is positioned perpendicularly between the at least one open end and the column body. Preferably, the mesh is positioned perpendicularly in the column body to the longitudinal axis of the column body.

Preferably, the binding of the cells is for the enrichment of the cells in the sample or for depletion of the cells from the sample.

Preferably, the non-laminar flow of the cells in the column body results in optimized binding of the cells compared to laminar flow thereof, wherein preferably the optimized binding is the enrichment of the cells in a sample and a higher cell purity and/or higher cell yield.

Preferably, the sample is whole blood, peripheral blood mononuclear cells or a cell therapeutic product.

Preferably, the cell is a blood cell, preferably a CD3+ blood cell or a NK cell. CD3 (cluster of differentiation 3) is a protein complex and T cell co-receptor that is involved in activating both the cytotoxic T cell (CD8+ naive T cells) and T helper cells (CD4+ naive T cells). More preferably, the biological entity is a CD4+ blood cell and/or CD8+ blood cell.

Preferably, a parallel system of the second aspect is provided, having at least 2 columns equipped with a stationary phase. After applying the sample to the sample filling unit, the cells bind to the stationary phases, preferably an affinity chromatography matrix for binding at least one epitope of the biological entity, comprised in column bodies of the at least 2 columns. The stationary phases are washed and the cells are eluted from the stationary phases and optionally removed from the system for further processing. Further processing may, for example, include additional washing steps or a genetic modification as mentioned above.

Preferably, the stationary phases of the at least 2 columns have a comparable binding affinity to the cells, e.g. to one selected epitope of the cells. In the alternative, the stationary phases of the at least 2 columns may preferably have different binding affinities and bind to different epitopes of cell or to different epitopes of different cells present in the sample.

The present invention also relates to a use of the system of the invention for binding a cell, preferably in a method of producing a cellular therapy product. The cell may be a blood cell, preferably a CD3+ blood cell or a NK cell, and more preferably, the biological entity is a CD4+ blood cell and/or CD8+ blood cell as mentioned above.

The invention relates in a third aspect to the column of the first aspect or the system of the second aspect for use in a method of *in vitro* diagnosis or in a method of producing a cellular therapy product.

Further, the invention relates to a method of *in-vitro* diagnosis or a method of producing a cellular therapy product, comprising the use of the column of the first aspect or the system of the second aspect.

The column of the first aspect or a system of the second aspect may be used in a method of producing a cellular therapy product. The column of the first aspect or a system of the second aspect may also be used for binding a cell. The column of the first aspect or a system of the second aspect may also be used in binding a cell of a sample for diagnostic or therapeutic purposes.

The invention provides in a fourth aspect a method of producing a cellular therapy product comprising the use of a column of the first aspect or a system of the second aspect for binding a cell.

Preferably, the column of the first aspect or a system of the second aspect is part of a tubing-set.

The tubing-set as used herein refers to a closed and preferably single-use tubing-set or a closed and preferably single-use cassette, e.g. for use in a method of manufacturing of a cellular therapy product. In the method of manufacturing of the cellular therapy product cells, preferably blood cells, are enriched in the sample, genetically modified for generating a precursor of the cellular therapy product, and formulated in a buffer for administering the precursor to a patient in which the cellular therapy product is generated.

The tubing-set may comprise a system for blood withdrawal, a system for reinfusion of cells into the human body, a target cell retention reservoir, a genetic modification unit, a target cell washing and formulation unit, a blood reservoir, one or more bag containing process buffers, a bag containing a set of reagents for gene modification a waste reservoir and a re-infusion reservoir, wherein the reservoirs and bags are fluidly connected by a closed tube system and wherein a target cell is a cell which is genetically modified within the method of manufacturing a cellular therapy product. The system of columns of the second aspect can, as part of the tubing set, be the target cell retention reservoir or be part of the target cell retention reservoir, the genetic modification unit, and/or the target cell washing and formulation unit. The tubing set may be used together with a device for operating and controlling the method.

The column of the first aspect or a system of the second aspect could be part of the target cell retention reservoir, the genetic modification unit, and/or the target cell washing and formulation unit. The tubing-set may be used together with a device for operating and controlling the method.

The present invention also relates in a fifth aspect to a method for binding a cell, the method comprising providing at least one column of the first aspect, adding a sample comprising the cell through the at least one open end of the column, and removing the cell from the sample. Optionally the at least one column is part of a system of the second aspect.

The complete disclosure of the first to fourth aspect is herein incorporated by reference.

Preferably, after adding the sample, the cell binds to a stationary phase, preferably an affinity chromatography matrix, comprised in the column body, the stationary phase is washed and the cell is eluted from the stationary phase and removed from the column via the at least one open end, wherein the at least one obstacle of the column body is arranged to achieve a non-laminar flow of the biological entity in the column body.

Preferably, the binding of the cell is for the enrichment of the cell or for depletion of the cell from a sample.

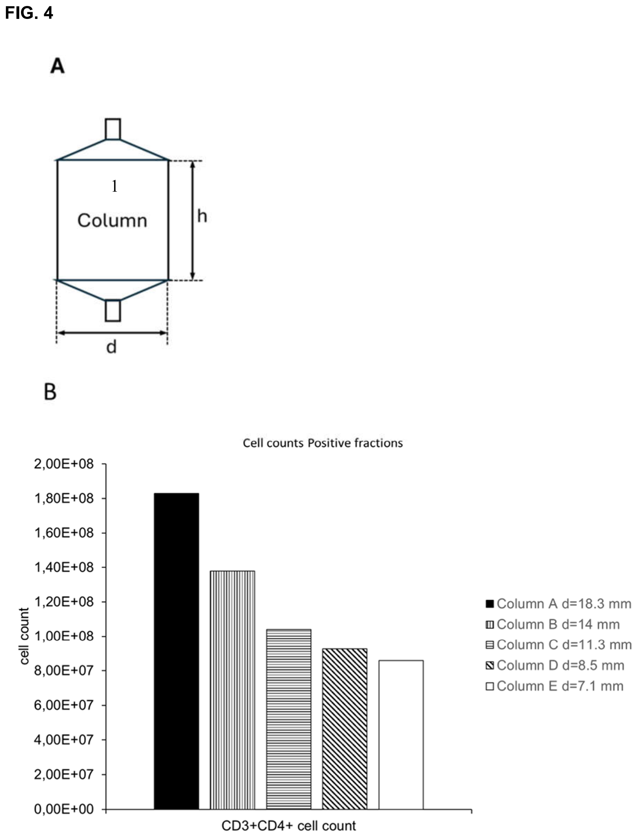

Preferably, the binding of the biological entity, which has a low concentration in the sample, to the matrix is enhanced by using a column, in particular a cylindrical column, with a small diameter, preferably between 11.1 mm and 7 mm, or with a ratio of column diameter to column body length of less than 0.1, or between about 0.1 and 0.05, compared to a cylindrical column with a higher diameter or a higher ratio of column diameter to column length, as also illustrated in Fig. 4. Preferably, the purity of the enriched biological entity is increased when using a column, in particular a cylindrical column, with a small diameter, preferably between about 11.1 mm and 7 mm, or with a ratio of column diameter to column body length of less than 0.1, or between about 0.1 and 0.05, compared to a cylindrical column with a higher diameter or a higher ratio of column diameter to column length. Preferably, the binding of the biological entity, which has a high concentration in the sample, to the matrix is enhanced by using a column, in particular a cylindrical column, with a wide diameter, preferably between about 19 mm and 11 mm, or with a ratio of column diameter to column body length of more than 0.1, or between about 1 and 0.1, compared to a cylindrical column with a lower diameter or a lower ratio of column diameter to column length.

Preferably, the sample comprises whole blood or peripheral blood mononuclear cells. Preferably, the cell is a blood cell, preferably a CD3+ blood cell or a NK cell, more preferably a CD4+ blood cell and/or CD8+ blood cell.

In an embodiment of the method of the fifth aspect, the system of the second aspect with a linear arrangement of columns as described above is provided, having at least 2 columns equipped with a stationary phase. After applying the sample to the sample filling unit, the cells bind to the stationary phases, preferably an affinity chromatography matrix for binding at least one epitope of the cell, comprised in the column bodies of the at least 2 columns, the stationary phases are washed and the cells are eluted from the stationary phases.

In an embodiment of the method of the fifth aspect, the system of the second aspect with a linear column arrangement as described above is provided, wherein at least one first column is equipped with a first stationary phase able to bind to the epitope of a first cell comprised in the sample and wherein at least one second column is equipped with a second stationary phase binding to the epitope of a second cell comprised in the sample. After applying the sample to the sample filing unit, the first cell binds to the first stationary phase of the at least one first column and the second cell binds to the second stationary phase of the at least one second column. After optional washing, optionally the first or second cell is used for further processing after specific elution. Further optionally the second cell is eluted from the second stationary phase and a combination of the first and second cells is used for further processing after elution of the cells.

In an embodiment of the method of the fifth aspect, the system of the second aspect with a linear arrangement of columns as described above is provided, wherein at least one first column is equipped with a first stationary phase able to bind to a first (target) epitope of the cell and wherein at least one second column is equipped with a second stationary phase binding to a second (target) epitope of the cell. The sample used in this method may comprise cells having the first and the second (target) epitopes, having only the first (target) epitope or optional having none of the first or second (target) epitopes. After applying the sample to the sample filing unit, the first epitope of the cell binds to the first stationary phase of the at least one first column. The cell is then eluted from the first stationary phase, optionally washed, and applied to the second column, wherein the second epitope of the cell binds to the second stationary phase. After optional washing the cell is eluted from the second column for further processing.

The cell, the first or the second cell are preferably a blood cell, preferably a CD3+ blood cell or a NK cell, more preferably a CD4+ blood cell and/or CD8+ blood cell(s).

The method of the fifth aspect may also be used in a method of producing a cellular therapy product. Thus, in an embodiment of the fourth aspect the invention relates to a method of producing a cellular therapy product comprising the method of the fifth aspect.

As mentioned elsewhere herein, the cellular therapy product may be, for example, a CAR T cell therapy product. Such CAR T cell therapy product may be used in treating cancer. Furthermore, the method of the fifth aspect may be used for diagnostic or therapeutic purposes. Accordingly, the method of the fifth aspect may be used to diagnose a disease, such as certain types of cancer, such as blood cancer, or infectious diseases. In such applications, target cells characteristic of the disease to be diagnosed may be selected for using the column and the method of the aspects of the present invention from the blood taken from a patient potentially suffering from the disease to be diagnosed. Furthermore, said disease may be treated by depleting said cells from the blood taken from the patient, which is then returned into the patient. Thus, the method of the fifth aspect could be used for *in-vitro* diagnosis or in a method of *in-vitro* diagnosis.

It must be noted that as used herein, the singular forms "a", "an" and "the" include plural references and vice versa unless the context clearly indicates otherwise. Furthermore, the term "cell" includes the singular and the plural unless the context clearly indicates otherwise. Thus, the term "cell" means a single cell and/or a plurality of cells. The term "first cell" and "second cell" means a first cell and/or a plurality or population of first cells and a second cell and/or a plurality or population of second cells.

Unless otherwise indicated, the term "at least" preceding a series of elements is to be understood to refer to every element in the series.

Those skilled in the art will recognize or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the claims, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms. E.g., the term "comprising" is meant to provide explicit support also for "consisting essentially of" and "consisting of", the term "consisting essentially of" is meant to provide explicit support also for "comprising" and "consisting of", the term "consisting of" is meant to provide explicit support also for "consisting essentially of" and "comprising". The possibility to replace terms with each other is not to be understood that these terms are synonymous.

The invention is further characterized by the following items:

1. A column for binding a cell, the column comprising:

   a column body comprising a retention volume;

   at least one open end for adding or removing the biological entity to or from the column; and

   at least one obstacle within the column body, wherein the at least one obstacle is a grid or a pin;

   optionally wherein the column has a first open end and a second open end arranged opposite to each other on the column.

2. The column of item 1, wherein the retention volume comprises a stationary phase, preferably an affinity chromatography matrix.

3. The column of items 1 or 2, wherein the pin is a protrusion in the column body, and wherein the pin is arranged at an angle between about 90° and about 100° to the longitudinal or transversal axis of the column; has a length between about 25% and about 100% of the column diameter; has a width between about 25% and about 50% of the column diameter; and has a thickness between about 2 mm and about 8 mm.

4. The column of items 1 or 2, wherein the grid comprises a surface, the surface is arranged at an angle between about 90° and about 100° to the longitudinal, transversal, and/or diagonal section of the column, wherein the surface extends across the column or a portion thereof, the surface comprising two or more perforations, and wherein the total area of the perforations is between about 25% and about 75% of the area of the surface, and wherein the grid has a thickness of about 1 mm to about 8 mm.

5. The column of any one of the preceding items, wherein the column comprises at least 2, at least 3, at least 4, at least 5, or at least 6 obstacles, wherein preferably the column comprises at least 2, at least 3, or at least 4 pins, wherein optionally each subsequent pin is arranged in the opposite direction and with an offset to a preceding pin, and wherein preferably the column comprises at least 2, at least 3, at least 4, at least 5, or at least 6 grids, wherein optionally the grids are arranged parallel to each other, preferably perpendicular to the column's longitudinal axis.

6. The column of any one of items 1 to 5, wherein the column body has the shape substantially of a sphere, capsule, ellipsoid, bicone, conical cylinder, biconical cylinder, cylinder, or a polyhedron.

7. The column of item 6, wherein the bicone, conical cylinder or biconical cylinder comprise truncated cones.

8. The column of any one of items 6 or 7, wherein the bicone or biconical cylinder comprise two cones with different dimensions.

9. The column of item 6, wherein the column body has the shape substantially of the cylinder and a ratio of a column body diameter to column body length is lower than 0.1, or is between 0.1 and 0.01.

10. The column of item 6, wherein the column body has the shape substantially of the cylinder and a ratio of a column body diameter to column body length is higher than 0.1, or is between 1 and 0.1.

11. The column of any one of the preceding items, wherein the column further comprises at least one mesh positioned perpendicularly between the at least one open end and the column body, and/or wherein at least one mesh is positioned in the column body perpendicularly to the longitudinal axis of the column body.

12. The column of item 11, wherein the at least one mesh is a mesh, preferably with a mesh size between about 5μm and about 50 μm.

13. The column of items 11 or 12, wherein at least one mesh is positioned between the first open end and the column body and between the second open end and the column body, wherein the at least one mesh is oriented perpendicularly to the longitudinal axis of the column body.

14. The column of any one of the preceding items, wherein the binding of the cell is for the enrichment of the cell in a sample or for depletion of the cell from a sample.

15. The column of any one of the preceding items, wherein a non-laminar flow of the cell in the column body results in optimized binding of the cell compared to laminar flow thereof.

16. The column of any one of the preceding items, wherein the cell is a blood cell, preferably a CD3+ blood cell, more preferably a CD4+ blood cell and/or CD8+ blood cell(s).

17. The column of any one of the preceding items, wherein the cell is an antigen expressing cell in a suitable buffer.

18. The column of one of the items 14 to 17, wherein the optimized binding is the enrichment of the cell in a sample and a higher cell purity and/or higher cell yield.

19. System of multiple columns for binding of a cell having one or more epitopes from a sample, comprising two or more columns, each column having a first open end functions as an inlet of the column and a second open end functions as an outlet of the column and a column body, wherein the column body comprises a retention volume, arranged in a line and/or in parallel, the system further comprises one or more pumps, a sample filling unit, and optionally a washing unit.

20. The system of item 19, wherein at least one of the columns is a column of any one of the items 1 to 18.

21. The system of items 19 or 20, wherein the columns are arranged in a line in that the outlet of one column is fluidly connected with the inlet of another column or that the outlet of the one column is fluidly connected with an inlet of a pump and an outlet of the pump is fluidly connected with the inlet of the other column or wherein the outlet of the pump is not connected with the inlet of the other column or wherein the outlet of the other column or the outlet of the pump is connected to a sample collecting unit.

22. The system of item 21, wherein the pump is arranged after every column or after every 2nd, 3rd, 4th, 5th, 6th or Nth column of the system, wherein the outlet of the last pump of the line of columns of the system is or is not connected with the inlet of the further column and wherein N is any natural number between 2 and 10.

23. The system of any one of items 21 or 22, wherein the sample filling unit has at least one fluid connection to the inlet of the first column of the line of columns for sample application, or wherein the sample filling unit is the inlet of the first column in the line of columns.

24. The system of any one of items 21 to 23, wherein the retention volume of at least one column comprises a stationary phase, preferably an affinity chromatography matrix for binding at least one epitope of the cell, and wherein the stationary phases of the columns have the same or different binding affinities.

25. The system of item 19, wherein the columns are each fluidly connected via their first open ends with the sample filling unit and arranged in parallel.

26. The system of item 25, wherein a group of columns is fluidly connected via their outlets with exactly one pump.

27. The system of item 25, wherein a group of columns is fluidly connected via their outlets with a group of pumps in that exactly one column is assigned to one pump.

28. The system of any one of items 25 to 27, wherein the system comprises the group of columns of item 26 or the group of columns of item 27 or a combination of both groups of columns.

29. The system of any one of items 25 to 28, wherein the retention volume of at least one column comprises a stationary phase, preferably an affinity chromatography matrix for binding at least one epitope of the cell, and wherein the stationary phases of the columns have the same or different binding affinities.

30. The system of any one of the items 19 to 29, wherein the system comprises 2, 3, 4, 5, 6, 7 or N columns, wherein N is any natural number between 2 and 10.

31. The system of any one of the items 19 to 30, wherein the system comprises at least two lines of columns wherein the columns of each line are arranged according to any one of items 21 to 24 having at least one pump in each line and wherein the at least two lines of columns are arranged in parallel according to any one of items 6 to 8 wherein one line of columns equals one column of any one of items 25 to 27 and wherein each inlet of the first column of each line is fluidly connected to the sample filling unit.

32. The system of item 31, wherein the number of lines of columns is between 2 and 10, or between 2 and 9, or between 2 and 8, or between 2 and 7, or between 2 and 6, or between 2 and 5, or between 2 and 4, or between 2 and 3, or wherein the number of series of columns is 2.

33. The system of one of the items 19 to 32, wherein the flow through the at least one column is uni-directional or bi-directional.

34. The system of one of the items 19 to 33, wherein the column body of the at least one column comprises at least one obstacle, wherein the at least one obstacle is arranged to achieve a non-laminar flow of the cell in the column body.

35. The system of item 34, wherein the at least one obstacle within the column body is a mesh, grid, or a pin, wherein preferably the at least one obstacle is positioned within the column body substantially perpendicularly, diagonally or horizontally to the longitudinal axis of the column body, wherein preferably the at least one obstacle extends at least across a portion of the column body or across the entire column body substantially perpendicularly, diagonally or horizontally to the longitudinal axis of the column body.

36. The system of any one of the items 19 to 35, wherein the binding of the cell is for the enrichment of the cell in the sample or for depletion of the cell from the sample.

37. The system of any one of the items 24 to 36, wherein the non-laminar flow of the cell in the column body results in optimized binding of the cell compared to laminar flow thereof, wherein preferably the optimized binding is the enrichment of the cell in a sample and a higher cell purity and/or higher cell yield.

38. The system of any one of the items 19 to 37, wherein the sample is whole blood, peripheral blood mononuclear cells or a cell therapeutic product.

39. The system of any one of the items 19 to 38, wherein the cell is a blood cell, preferably a CD3+ blood cell or a NK cell, more preferably a CD4+ blood cell and/or CD8+ blood cell.

40. The column of any one of items 1 to 18 or the system of any one of the items 19 to 39 to be used in a method of producing a cellular therapy product.

41. The column of any one of items 1 to 18 or the system of any one of the items 19 to 39 for use in binding a cell of a sample for diagnostic or therapeutic purposes.

42. The column of any one of items 1 to 18 or the system of any one of the items 19 to 39 for use in binding a cell of a sample for *in-vitro* diagnosis.

43. A method of *in-vitro* diagnosis comprising a use of the column of any one of items 1 to 18 or the system of any one of the items 19 to 39.

44. A method of producing a cellular therapy product comprising the use of the column of any one of items 1 to 18 or the system of any one of the items 19 to 39 for binding a cell.

45. The method of item 44, wherein the column or the system is part of a tubing set, preferably wherein the column is part of a target cell retention reservoir, a genetic modification unit, and/or a target cell washing and formulation unit of the tubing set.

46. A method for binding a cell, the method comprising:

providing at least one column of any one of items 1 to 18;

adding a sample comprising the cell through the at least one open end of the at least one column; and

removing the enriched cell from the sample.

47. The method of item 46, wherein the at least one column is part of a system of any one of claims 19 to 39.

48. The method of items 46 or 47, wherein, after adding the sample, the cell binds to a stationary phase, preferably an affinity chromatography matrix, comprised in the column body, the stationary phase is washed and the cell is eluted from the stationary phase and removed from the column via the at least one open end, wherein the at least one obstacle of the column body is arranged to achieve a non-laminar flow of the cell in the column body.

49. The method of any one of items 46 to 48, wherein the binding of the cell is for the enrichment of the cell in a sample or for depletion of the cell from a sample.

50. The method of any one of items 46 to 49, wherein the binding of the cell, which has preferably a low concentration in the sample, to the matrix is enhanced by using a column, in particular a cylindrical column, with a small diameter, preferably between 11.1 mm and 7 mm, or with a ratio of column diameter to column body length of less than 0.1, or between about 0.1 and 0.05, compared to a cylindrical column with a higher diameter or a higher ratio of column diameter to column length.

51. The method of any one of items 46 to 50, wherein the purity of the enriched cell is increased when using a column, in particular a cylindrical column, with a small diameter, preferably between about 11.1 mm and 7 mm, or with a ratio of column diameter to column body length of less than 0.1, or between about 0.1 and 0.05, compared to a cylindrical column with a higher diameter or a higher ratio of column diameter to column length.

52. The method of any one of items 46 to 51, wherein the binding of the cell, which has preferably a high concentration in the sample, to the matrix is enhanced by using a column, in particular a cylindrical column, with a wide diameter, preferably between about 19 mm and 11 mm, or with a ratio of column diameter to column body length of more than 0.1, or between about 1 and 0.1, compared to a cylindrical column with a lower diameter or a lower ratio of column diameter to column length.

53. The method of any of items 46 to 52, wherein the sample comprises whole blood or peripheral blood mononuclear cells.

54. The method of any one of items 46 to 53, wherein the cell is a blood cell, preferably a CD3+ blood cell or a NK cell, more preferably a CD4+ blood cell and/or CD8+ blood cell(s).

55. The method of any one of items 47 to 54, wherein a system according to any one of items 19 to 39 is provided, having at least 2 columns equipped with a stationary phase, wherein the cell, after applying the sample to the sample filling unit, binds to the stationary phases, preferably an affinity chromatography matrix for binding at least one epitope of the cell, comprised in column bodies of the at least 2 columns, the stationary phases are washed and the cell is eluted from the stationary phases and optionally removed from the system for further processing.

56. The method of item 55, wherein the stationary phases of the at least 2 columns have a comparable binding affinity to the cell.

57. The method of item 55, wherein the stationary phases of the at least 2 columns having different binding affinities and bind to different epitopes of one cell or to epitopes of different cells present in the sample.

58. The method of any one of items 47 to 54, wherein a system according to any one of items 20 to 25 or 30 to 39 is provided, having at least 2 columns equipped with a stationary phase, wherein the cell, after applying the sample to the sample filling unit, binds to the stationary phases, preferably an affinity chromatography matrix for binding at least one epitope of the cell, comprised in the column bodies of the at least 2 columns, the stationary phases are washed and the cell is eluted from the stationary phases.

59. The method of any one of items 47 to 54, wherein a system according to any one of items 20 to 25 or 30 to 39 is provided, wherein at least one first column is equipped with a first stationary phase able to bind to the epitope of a first cell comprised in the sample and wherein at least one second column is equipped with a second stationary phase binding to the epitope of a second cell comprised in the sample, wherein, after applying the sample to the sample filing unit, the first cell binds to the first stationary phase of the at least one first column and the second cell binds to the second stationary phase of the at least one second column, after optional washing, optionally the first or second cell is used for further processing after specifically elution or further optional the second cell is eluted from the second stationary phase and a combination of the first and second cells is used for further processing after elution of the biological entities.

60. The method of any one of items 47 to 54, wherein a system according to any one of items 20 to 25 or 30 to 39 is provided, wherein at least one first column is equipped with a first stationary phase able to bind to a first epitope of the cell and wherein at least one second column is equipped with a second stationary phase binding to a second epitope of the cell, wherein the sample comprises cells having the first and the second epitope, having only the first epitope or optional having none of the first or second epitope, wherein, after applying the sample to the sample filing unit, the first epitope of the cell binds to the first stationary phase of the at least one first column, the cell is eluted from the first stationary phase, optional washed, and applied to the second column, wherein the second epitope of the cell binds to the second stationary phase and after optional washing the cell is eluted from the second column for further processing.

61. The method of any one of items 46 to 60 to be used in a method of producing cellular therapy product.

62. The method of any one of items 46 to 60 for use in diagnosis or therapy of a disease.

63. A method of in-vitro diagnosis comprising the method of any one of items 46 to 60.

64. A method of diagnosis or treatment of a disease comprising the method of any one of items 46 to 60.

**EXAMPLES**

**Example 1: Enrichment of CD3+ cells**

CD3+ cells, which can be used as starting cells for a cellular therapy product, such as CAR T cells, were selectively and reversibly immobilized from a whole blood sample.

*Production of the anti-CD3 affinity matrix for reversible immobilization of cells*

For the enrichment of CD3+ cells, i.e. the target cells, via reversible immobilization, an anti-CD3 affinity matrix was used. The matrix composition is described in the following (for 20 mL matrix). 10 mg of multimerized streptavidin mutein (Strep-Tactin® m2; described in US 6,103,493) was coupled to 8 g dry weight epoxy-activated polystyrene resin (CY17030; Cytosorbents) using solvent-exposed primary amine groups, in phosphate buffered saline (PBS) buffer. Afterwards, 1.8 mg of target cells selection agent with an anti-CD3 Fab fragment and a streptavidin-binding peptide (Twin-Strep-tag®, Twin-Strep-tag® streptavidin-binding peptide including a sequential arrangement of two streptavidin-binding modules) carboxyl-terminally fused to the heavy chain of the Fab fragment, was added to the Strep-Tactin®-coated 1:1 matrix suspension (total volume vs. matrix bed volume). The matrix suspension was incubated under constant gentle movement at room temperature for 60 min to enable the reversible binding of the anti-CD3 agent to the matrix via the immobilized Strep-Tactin®. 20mL final anti-CD3 functionalized matrix were filled into a reservoir bigger than 20mL to enable movement of the matrix during blood loading, washing of the non-target cells and release of the enriched target cells.

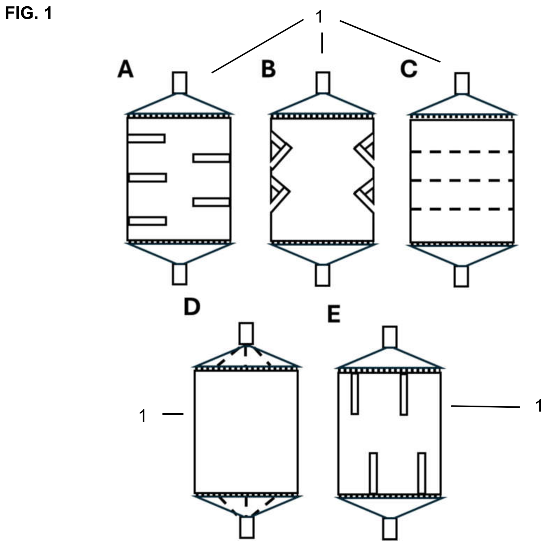

Prior to enriching the target cells, the reservoir including the matrix was equilibrated with wash buffer (PBS with 0.5% human serum albumin (HSA)). The above-mentioned anti-CD3 Fab fragment is peptide-tagged and was produced recombinantly (see WO 2013/011011 and WO 2013/124474). The Fab fragment was derived from the CD3 binding monoclonal antibody produced by the hybridoma cell line OKT3 (ATCC® CRL-8001 ™; see also US 4,361,549). It contains the heavy chain variable domain and light chain variable domain of the anti-CD3 antibody OKT3 e.g. described in figure 1 in Arakawa et al., 1996.

*Enrichment process of target cells*

200 mL of whole blood from a healthy human donor with anticoagulant were transferred into one bag. Subsequently, the bag was connected to a tubing set and the blood was flushed stepwise into the column 1 of the invention containing anti-CD3 affinity matrix (prepared as describe above) where the target cells, e.g. for use as a cellular therapy starting cells, are immobilized for a short time while non-target cells flow through the affinity matrix and out of the column 1. Blood loading was performed at 20 mL per minute (calculated with a standard blood tubing) in 15 mL pulses, following a reverse-pulse and incubation of blood under gentle moving conditions of the matrix particles after each 15 mL pulse. After complete loading of 200 mL whole blood, non-target cells were washed out with wash buffer (PBS with 0,5% HSA) at 40 mL per minute (calculated with a standard blood tubing) in 40 mL pulses using a reverse pulse after each wash step and under gentle movement of the matrix. In total, 9 washing pulses were performed. After the washout of the non-target cells, the short enrichment step of target cells was finished by flushing a 100 μM D-Biotin buffer solution (PBS with 0,5% HSA and 100 μM D-Biotin) into the matrix.

Target cell release was performed at 75 mL per minute (calculated with a standard blood tubing) in 30 mL pulses with a reverse pulse after each step, in total 3 times under gentle movement of the matrix. Further target cell release was performed by flushing the matrix with 30 mL D-Biotin buffer followed by an incubation step with stronger movement of the matrix for 3 min, repeating this release procedure 7 times. Thus, the target cells were specifically enriched by a reversible immobilization using the anti-CD3 affinity matrix.

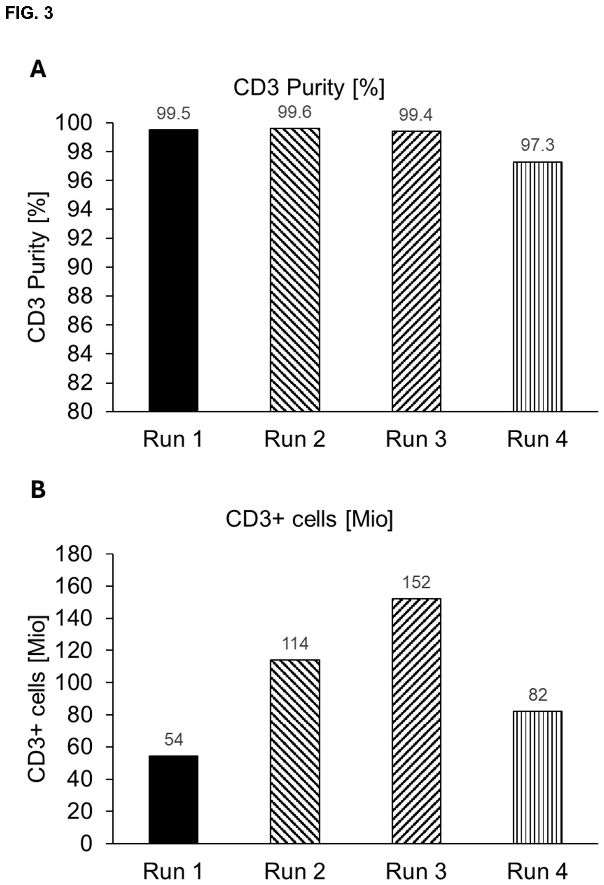

Fig. 3A and B show the cell count and purity of the enriched target cells, which can be used e.g. as starting cells for the production of cellular therapy products, e.g. CAR T cells.

**Example 2: Effect of different column geometries on cell purity**

Selected designs of columns 1 were tested for CD4+ T cell selection capability out of apheresis. The different columns 1 to 5 as shown in Fig. 4A with the dimensions listed in Table 1 were printed with a 3D printer (Form 2 by Formlabs). One column body and two column caps were printed and glued together to produce one column 1. A double 20 μm polyamide mesh was inserted between the bottom cap and the column body to provide non-laminar flow and to hold the resin in the column 1.

Table 1

|  | d (mm) | h (mm) |
|---|---|---|
| Column A | 18.3 | 22.5 |
| Column B | 14 | 55 |
| Column C | 11.3 | 60 |
| Column D | 8.5 | 105 |
| Column E | 7.1 | 150 |

The effect of the different column 1 geometries on cell selection was evaluated by comparing the performance of CD4+ cell selection from PBMCs. A peristaltic pump 2 was used and the volumetric flow rate of the peristaltic pump 2 was kept constant, thus resulting in proportionally higher or lower flow rates through the column 1. A syringe pump 2 could be used as an alternative. The performance was investigated for the following parameters: purity of target cell fraction (CD3+CD4+), cell count of target cell fraction in the positive fraction (CD3+CD4+), cell count of target cell fraction bound to the matrix (CD3+CD4+), and the amount of monocytes in the positive fraction. Results are summarized in Table 2 and Fig.

4B.

Table 2

| Column 1 (as shown in Fig. 4A and Table 1) | Purity CD4+ (%) | Purity CD3+CD4+ (%) |
|---|---|---|
| Column A (d=18.3 mm | 97 | 93 |
| Column B (d=14 mm) | 99 | 95 |
| Column C (d=11.3 mm) | 98 | 96 |
| Column D (d=8.5 mm) | 98 | 96 |
| Column E (d=7.1 mm) | 98 | 96 |

Column designs 1 with a wider diameter and therefore a lower flow rate feature an increased binding of target cells as can be seen from Fig. 4B. However, due to the favourable binding conditions in the case of the column 1 with a wider diameter, low expressing CD4+ cells (in this case monocytes) also increased binding to the matrix, and thus purity of the target cells (CD3+CD4+) was lower (see Table 1). Accordingly, smaller diameter column 1 leads to higher purity of target cells but also to concomitant lower cell counts. Thus, smaller diameter seems to be better suited for selecting low expressing antigen target cells or target cells with a lower overall concentration in the starting material (like PBMCs, apheresis or blood). Wider diameter seems to be better for selecting high expressing antigen target cells, or cells with high concentrations in the starting material, leading to higher cell counts but also to increased binding of non-wanted target cells. Therefore, small diameter of the column leads to higher purity of CD3+CD4+ target cells and to lower cell count, and wide diameter of the column leads to lower purity of CD3+CD4+ target cells and to higher cell count.

**Example 3: Non-laminar flow through columns comprising obstacles.**

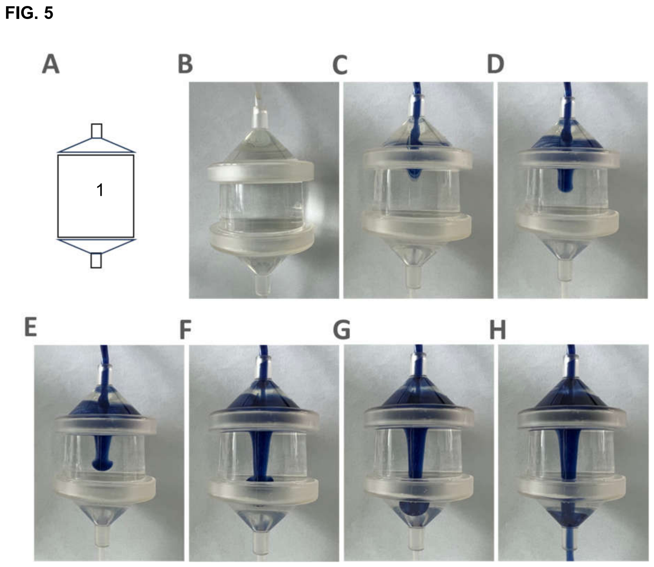

The effect of the obstacles in the column 1 of the present invention on the flow of a liquid through the column 1 was investigated. First, the inventors postulated that a column 1 not comprising any obstacles would feature a highly laminar flow with uneven flow rates through the column 1. The inventors therefore conducted an experiment wherein a column 1 without obstacles was filled with water and coloured water was flushed through the column 1 with a pump at a speed of 30 ml/min. As expected, the flow through the column without obstacles was laminar, with the flow concentrated in the centre of the column 1 (see Fig. 5).

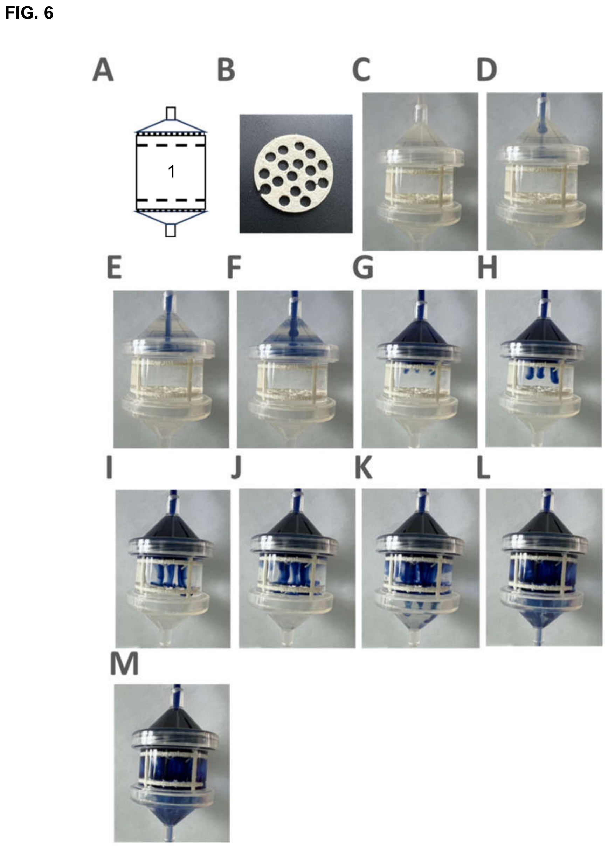

Next, the inventors investigated a column 1 of the invention comprising two grids as obstacles. As shown in Fig. 6A, the grids were positioned towards both ends of the column 1. In this embodiment, each grid comprised 17 holes in the grid surface, the diameter of each hole being approximately 14% of the diameter of the grid's surface, i.e. inner diameter of the column body (Fig. 6B). Accordingly, in this example the sum of the hole surface in each grid was approximately 27% of the surface of the grid face. As shown in Fig. 6C-M, the flow through the column 1 comprising two grids was non-laminar. The whole retention volume of the column body was filled with the incoming coloured fluid in an even manner, as a result of the turbulent flow achieved by the obstacles, such as grids, positioned in the column body.

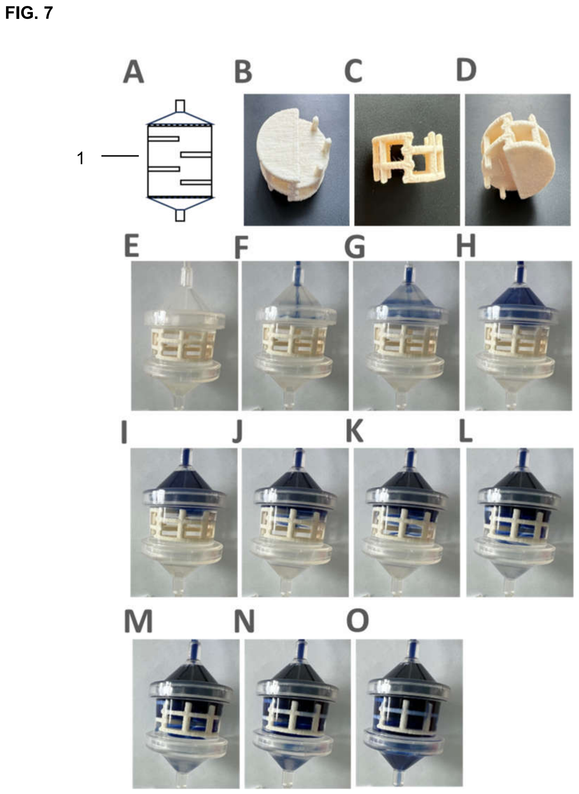

Next, the inventors investigated a column 1 of the invention comprising four pins as obstacles. As shown in Fig. 7A, pins were positioned opposite one another with an offset, the offset chosen adequately to distribute the pins evenly across the longitudinal axis of the column 1. The pins were directed at 90° angle to the longitudinal axis of the column body, had a length approximately equal to the inner diameter of the column body, and had a width approximately 50% of the inner diameter of the column body (Fig. 7B-D). In this example, the obstacles (i.e. pins) were 3D printed as a single object, including vertical support structures. The single object comprising the pins may be inserted into a column 1. As shown in Fig. 7E-O, the flow through the column 1 comprising four pins was non-laminar. The whole retention volume of the column body was filled with the incoming coloured fluid in an even manner, as a result of the turbulent flow achieved by the obstacles, such as pins, positioned in the column body.

**Example 4: Arrangement of the columns in the system of the invention**

Any number of columns 1 may be used in the system of the invention to perform the method of the invention.

Figure 8A shows an example serial configuration of four columns 1 and a single pump 2, that can be used in selection and/or depletion (negative selection) of target cells. All the columns 1 may be the same, some of the columns 1 may be different or all columns 1 may be different in terms of size, shape, and selection affinity matrix. The configuration here may be used with identical columns 1 to select the same target cell from the same starting material to increase the target cell binding capacity. Alternatively, each column 1 in this configuration may be configured to select different target cells from the same starting material, for example a combination of different cells (also in a specific cell count ratio according to used column size) for further cell processing and manufacturing a cell therapy, or to select several target cells with the objective of depleting unwanted cells from the sample.

This configuration may be advantageous for selecting target cells from the flow-through of the previous column 1, the cell counts determined by the column size. As an illustrative example, starting sample may be peripheral blood mononuclear cells (PBMC), from which specific unwanted cells are bound to the first column 1 and the flow through is used to feed the second column 1. Starting sample may also be a final cell product, wherein specific unwanted cells (e.g. CD3+) are bound to the column 1 and the flow-through is free of CD3+ cells. Starting sample may also be whole blood and the first column 1 may be a platelet binding column 1 (with non-reversible antibodies), thereby depleting platelets and leading to a better binding of T cells to the subsequent columns 1; the second column 1 may select e.g. CD3+ cells.

Figure 8B shows an example series configuration of three columns 1, one wash unit 3, and one pump 2. The columns 1 may be the same or different. This configuration can be applied for stepwise depletion of the sample, selection of target cells from the depleted sample prior to the wash unit 3, subsequent elution and washing of target cell fraction, and selection of a subset of target cells from the previously washed target cells. This configuration is advantageous for obtaining more specialized cells (e.g. CD3+CD4+, CD3+CD8+, CD4+CD25+) for further cell processing and manufacturing of a cell therapy product.

An alternative configuration to the above is shown in Figure 8C, which depicts an example series configuration of two columns 1, one wash unit 3, and two pumps 2. The columns 1 may be the same or different. This configuration allows the selection of different target cells from the same starting material by selecting first population of target cells, then eluting and washing of the first target cell population, and selecting a second population of target cells from the first population of targets cells, wherein the second population is a subset of the first population. The advantage of two pumps 2 here is the ability to set different pump speeds for each column 1, to accommodate different optimal speeds/binding conditions per column 1 to achieve optimal purities and yields. Thus, such serial configuration arrangement of different columns 1, as shown in Figure 8B and C, allows to select specialized cells from a larger general population of cells.

Figure 9A shows an example parallel configuration of four columns 1 operated by four pumps 2. The columns 1 may be the same or different. This configuration allows to select the same target cell or different target cells from the same sample in a defined cell count ratio (owing to different amount of matrix and/or different pump speed operating each column 1) to be used e.g. for analysis and analytic assays, for example a target cell selection for further cell processing in parallel to sample analysis. Here, target cells can be obtained in different bags for different purposes, the selection process of each column 1 may be shifted in time (owing to one pump 2 per column 1 allowing independent operation) when aiming for different cell counts and purities. This parallel configuration may be used to increase target cell binding capacity and to decrease target cell selection time with more identical columns 1. By selecting different target cells, a ratio of different cell types present in a cell eluate for producing e.g. a cellular therapy product could be determined and adjusted to a preferred ratio.

Figure 9B shows an example parallel configuration of four columns 1 operated by one pump 2. The columns 1 may be the same or different. If identical columns 1 are used, this configuration is advantageous for the selection of the same target cell from the same sample, to increase target cell binding capacity, and to decrease target cell binding time. If different columns 1 are used, this configuration is advantageous for the selection of different target cells from the same starting material to achieve a combination of different target cells for further cell processing and manufacturing of a cell therapy product.

Figure 10A shows an example of a combined series and parallel configuration of four columns 1 operated by two pumps 2. The columns 1 arranged in any of the lines or series of the series-parallel configuration may be the same or different, and the column lines may each comprise any number of pumps 2. If identical columns 1 are used in the first line, this configuration allows for selection of the same target cell from the same starting material to increase target cell binding capacity with more columns. If different columns 1 are used in the line, this configuration is advantageous for depletion and selection of different target cells from the same starting material, in order to achieve e.g. a combination of different cells (in e.g. defined cell counts according to used column size) for further cell processing and manufacturing a cell therapy product, and depleting unwanted cells in the target cell material. The second line arranged in parallel with the first line may comprise any number of identical or different columns 1 to achieve a desired final cell selection. The second line arranged in parallel with the first line may also comprise a wash unit 3 (Figure 3B), thereby allowing selection of different subsequent target cells (selection of one cell marker, then elution and washing of target cell fraction and selection of next cell marker out of the previously washed target cell fraction) to obtain more specialized cells (e.g. CD3+CD4+, CD3+CD8+, CD4+CD25+) for further cell processing and manufacturing of a cell therapy.

It is understood that there may be more than two lines or series arranged in parallel, each comprising any arrangements described herein or derivable from the present disclosure.

## REFERENCES

Arakawa, F., Kuroki, M., Kuwahara, M., Senba, T., Ozaki, H., Matsuoka, Y., Misumi, Y., Kanda, H., Watanabe, T., 1996. Cloning and sequencing of the VH and V kappa genes of an anti-CD3 monoclonal antibody, and construction of a mouse/human chimeric antibody. J Biochem 120, 657-662. https://doi.org/10.1093/oxfordjournals.jbchem.a021462

Locke, F.L., Malik, S., Tees, M.T., Neelapu, S.S., Popplewell, L., Abramson, J.S., McDevitt, J.T., Shin, C.R., Demirhan, E., Konto, C., Lekakis, L.J., 2021. First-in-human data of ALLO-501A, an allogeneic chimeric antigen receptor (CAR) T-cell therapy and ALLO-647 in relapsed/refractory large B-cell lymphoma (R/R LBCL): ALPHA2 study. JCO 39, 2529-2529. https://doi.org/10.1200/JC0.2021.39.15_suppl.2529

Perez, C., Gruber, I., Arber, C., 2020. Off-the-Shelf Allogeneic T Cell Therapies for Cancer: Opportunities and Challenges Using Naturally Occurring "Universal" Donor T Cells. Front Immunol 11, 583716. https://doi.org/10.3389/fimmu.2020.583716

Xin, T., Cheng, L., Zhou, C., Zhao, Y., Hu, Z., Wu, X., 2022. In-Vivo Induced CAR-T Cell for the Potential Breakthrough to Overcome the Barriers of Current CAR-T Cell Therapy. Front. Oncol. 12. https://doi.org/10.3389/fonc.2022.809754

## Claims

1. A column for binding a cell, the column (1) comprising:

   a column body comprising a retention volume comprising a stationary phase, wherein preferably the stationary phase is an affinity chromatography matrix; at least one open end for adding or removing the cell to or from the column (1); and
   at least one obstacle within the column body, wherein the at least one obstacle is a grid or a pin;
   optionally wherein the column (1) has a first open end and a second open end arranged opposite to each other on the column (1).

2. The column of claim 1, wherein the pin is a protrusion in the column body, and wherein the pin is arranged at an angle between about 90° and about 100° to the longitudinal or transversal axis of the column; has a length between about 25% and about 100% of the column diameter; has a width between about 25% and about 50% of the column diameter; and has a thickness between about 2mm and about 8 mm.

3. The column of claim 1, wherein the grid comprises a surface, the surface is arranged at an angle between about 90° and about 100° to the longitudinal, transversal, and/or diagonal section of the column (1), wherein the surface extends across the column (1) or a portion thereof, the surface comprising two or more perforations, and wherein the total area of the perforations is between about 25% and about 75% of the area of the surface, and wherein the grid has a thickness of about 1 mm to about 8 mm.

4. The column of any one of the preceding claims, wherein the column (1) comprises at least 2, at least 3, at least 4, at least 5, or at least 6 obstacles, wherein preferably the column (1) comprises at least 2, at least 3, or at least 4 pins, wherein optionally each subsequent pin is arranged in the opposite direction and with an offset to a preceding pin, and wherein preferably the column (1) comprises at least 2, at least 3, at least 4, at least 5, or at least 6 grids, wherein optionally the grids are arranged parallel to each other, preferably perpendicular to the column's longitudinal axis.

5. The column of any one of the preceding claims, wherein the column body has the shape substantially of a sphere, capsule, ellipsoid, bicone, conical cylinder, biconical cylinder, cylinder, or a polyhedron, wherein preferably the bicone, conical cylinder or biconical cylinder comprise truncated cones, wherein preferably the bicone or biconical cylinder comprises two cones with different dimensions.

6. The column of any one of the preceding claims, wherein the column (1) further comprises at least one mesh positioned perpendicularly between the at least one open end and the column body, and/or wherein at least one mesh is positioned in the column body perpendicularly to the longitudinal axis of the column body.

7. The column of any one of the preceding claims, wherein the binding of the cell is for the enrichment of the cell in a sample or for depletion of the cell from a sample.

8. The column of any one of the preceding claims, wherein the cell is a blood cell, preferably a CD3+ blood cell, more preferably a CD4+ blood cell and/or CD8+ blood cell.

9. A system for binding of a cell from a sample comprising two or more columns (1) arranged in a line and/or in parallel, the system further comprises one or more pumps (2), a sample filling unit, and optionally a washing unit (3), wherein at least one column is a column of any one of claims 1 to 8.

10. The column of any one of claims 1 to 8 or the system of claim 9 for use in a method of in vitro diagnosis or in a method of producing a cellular therapy product.

11. A method of producing a cellular therapy product comprising the use of a column (1) of any one of claims 1 to 8 or a system of claim 9 for binding a cell.

12. The method of claim 11, wherein the column (1) or the system is part of a tubing set, preferably wherein the column (1) or the system is part of a target cell retention reservoir, a genetic modification unit, and/or a target cell washing and formulation unit of the tubing set.

13. A method for binding a cell, the method comprising:

providing at least one column (1) of any one of claims 1 to 8;
adding a sample comprising the cell through the at least one open end of the at least one column (1);
optionally washing the stationary phase; and
removing the cell from the sample;
optionally wherein the at least one column (1) is part of a system of claim 9.

14. The method of claim 13, wherein the sample comprises whole blood or peripheral blood mononuclear cells, wherein preferably the cell is a blood cell, preferably a CD3+ blood cell or a NK cell, more preferably a CD4+ blood cell and/or CD8+ blood cell.

15. The method of claim 13 or 14 for use in a method of in vitro diagnosis or in a method of producing a cellular therapy product.

**FIG. 1**

**FIG. 2**

**FIG. 3**

**A**

CD3 Purity [%]

**B**

CD3+ cells [Mio]

# FIG. 4

A

B

Cell counts Positive fractions

FIG. 5

**FIG. 6**

**FIG. 7**

**FIG. 8**

**FIG. 9**

FIG. 10

| | Europäisches Patentamt European Patent Office Office européen des brevets | **EUROPEAN SEARCH REPORT** | **Application Number** EP 25 20 6964 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/178142 A1 (GJERDE DOUGLAS T [US]) 28 June 2018 (2018-06-28) | 1,3-15 | INV. B01D15/10 |
| Y | * claim 1 * | 11-15 | B01D15/18 |
| | * claim 4 * | | B01D15/22 |
| | * figures 3,8 * | | B01D15/38 |
| | * paragraph [0058] * | | C07K1/22 |
| | * paragraph [0032] - paragraph [0036] * | | B01L3/00 |
| | * claim 14 * | | G01N30/60 |
| | * figure 12 * | | |
| | * paragraph [0183] * ----- | | |
| X | WO 2016/100447 A1 (WATER TECHNOLOGIES CORP [US]) 23 June 2016 (2016-06-23) | 1,5,7,8, 10,11,13 | |
| Y | * claim 6 * | 11-15 | |
| | * claim 1 * | | |
| | * claims 39-40 * | | |
| | * line 38; example 2 * | | |
| | * claim 36 * | | |
| | * figure 2 * | | |
| | * table 3 * ----- | | TECHNICAL FIELDS SEARCHED (IPC) |
| X | WO 2022/076543 A1 (PHYNEXUS INC [US]) 14 April 2022 (2022-04-14) | 1,4-8, 10,11, 13,14 | B01D G01N |
| | * claim 1 * | | C07K |
| | * figure 1 * | | B01L |
| | * claim 31 * | | |
| | * example 4 * ----- | | |
| X | CN 210 543 500 U (YUNNAN HANMENG PHARMACEUTICAL CO LTD) 19 May 2020 (2020-05-19) | 1,2,4, 6-10 | |
| Y | * figure 1 * | 11-15 | |
| | * figure 2 * | | |
| | * claim 1 * ----- -/-- | | |

The present search report has been drawn up for all claims

| Place of search | Date of completion of the search | Examiner |
|---|---|---|
| Munich | 25 February 2026 | Oikonomou, Evdokia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 25 20 6964

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 522 172 A (PRETORIUS VICTOR ET AL) 28 July 1970 (1970-07-28) | 1,2,4-10 | |
| Y | * claim 20 * <br> * figures 3-8 * <br> ----- | 11-15 | |

TECHNICAL FIELDS
SEARCHED       (IPC)

The present search report has been drawn up for all claims

| Place of search | Date of completion of the search | Examiner |
|---|---|---|
| Munich | 25 February 2026 | Oikonomou, Evdokia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................
& : member of the same patent family, corresponding document

EPO FORM 1503 03.82 (P04C01)

Europäisches
Patentamt

European
Patent Office

Office européen
des brevets

**Application Number**

**EP 25 20 6964**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION**
**SHEET B**

The Search Division considers that the present European patent application does not comply with the requirements of unity of invention and relates to several inventions or groups of inventions, namely:

```
1. claims: 3(completely); 1, 4-15(partially)

   A column for binding a cell, the column (1) comprising: a
   column body comprising a retention volume comprising a
   stationary phase, wherein preferably the stationary phase is
   an affinity chromatography matrix;
   at least one open end for adding or removing the cell to or
   from the column (1); and at least one obstacle within the
   column body, optionally wherein the column (1) has a first
   open end and a second open end arranged opposite to each
   other on the column (1),
   wherein the at least one obstacle is a grid
                         ---


2. claims: 2(completely); 1, 4-15(partially)

   A column for binding a cell, the column (1) comprising: a
   column body comprising a retention volume comprising a
   stationary phase, wherein preferably the stationary phase is
   an affinity chromatography matrix;
   at least one open end for adding or removing the cell to or
   from the column (1); and at least one obstacle within the
   column body, optionally wherein the column (1) has a first
   open end and a second open end arranged opposite to each
   other on the column (1),
   wherein the at least one obstacle is a pin
                         ---
```

## ANNEX TO THE EUROPEAN SEARCH REPORT
## ON EUROPEAN PATENT APPLICATION NO.

EP 25 20 6964

This annex lists the patent family members relating to the patent documents cited in the above-mentioned European search report.
The members are as contained in the European Patent Office EDP file on
The European Patent Office is in no way liable for these particulars which are merely given for the purpose of information.

25-02-2026

| Patent document cited in search report | Publication date | Patent family member(s) | Publication date |
|---|---|---|---|
| US 2018178142 A1 | 28-06-2018 | US 2018178142 A1 | 28-06-2018 |
| | | US 2019358562 A1 | 28-11-2019 |
| WO 2016100447 A1 | 23-06-2016 | CN 107427742 A | 01-12-2017 |
| | | CN 119367815 A | 28-01-2025 |
| | | EP 3234585 A1 | 25-10-2017 |
| | | EP 3660503 A1 | 03-06-2020 |
| | | US 2018001229 A1 | 04-01-2018 |
| | | US 2022032210 A1 | 03-02-2022 |
| | | WO 2016100447 A1 | 23-06-2016 |
| WO 2022076543 A1 | 14-04-2022 | AU 2021357799 A1 | 04-05-2023 |
| | | CA 3191983 A1 | 14-04-2022 |
| | | CN 116323002 A | 23-06-2023 |
| | | IL 301577 A | 01-05-2023 |
| | | JP 2023545800 A | 31-10-2023 |
| | | KR 20230079220 A | 05-06-2023 |
| | | US 2023408383 A1 | 21-12-2023 |
| | | WO 2022076543 A1 | 14-04-2022 |
| CN 210543500 U | 19-05-2020 | NONE | |
| US 3522172 A | 28-07-1970 | DE 1673011 A1 | 10-08-1972 |
| | | GB 1183833 A | 11-03-1970 |
| | | US 3522172 A | 28-07-1970 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 24205275 A
- EP 24205282 A
- WO 2019217964 A1
- WO 2022072885 A1
- WO 2023122277 A2
- US 2018178142 A1

- WO 2016100447 A1
- US 6103493 A
- WO 2013011011 A
- WO 2013124474 A
- US 4361549 A

### Non-patent literature cited in the description

- **ARAKAWA, F** ; **KUROKI, M** ; **KUWAHARA, M.** ; **SENBA, T** ; **OZAKI, H** ; **MATSUOKA, Y** ; **MISUMI, Y.** ; **KANDA, H.** ; **WATANABE, T.** Cloning and sequencing of the VH and V kappa genes of an anti-CD3 monoclonal antibody, and construction of a mouse/-human chimeric antibody. *J Biochem*, 1996, vol. 120, 657-662, https://doi.org/10.1093/oxfordjournals.jbchem.a021462
- **LOCKE, F.L** ; **MALIK, S.** ; **TEES, M.T** ; **NEELAPU, S.S.** ; **POPPLEWELL, L** ; **ABRAMSON, J.S** ; **MCDEVITT, J.T.** ; **SHIN, C.R** ; **DEMIRHAN, E.** ; **KONTO, C.** First-in-human data of ALLO-501A, an allogeneic chimeric antigen receptor (CAR) T-cell therapy and ALLO-647 in relapsed/refractory large B-cell lymphoma (R/R LBCL): ALPHA2 study. *JCO*, 2021, vol. 39, 2529-2529, https://doi.org/10.1200/JC0.2021.39.15_suppl.2529

- **PEREZ, C.** ; **GRUBER, I** ; **ARBER, C.** Off-the-Shelf Allogeneic T Cell Therapies for Cancer: Opportunities and Challenges Using Naturally Occurring ''Universal'' Donor T Cells. *Front Immunol*, 2020, vol. 11, 583716, https://doi.org/10.3389/fimmu.2020.583716
- **XIN, T** ; **CHENG, L.** ; **ZHOU, C** ; **ZHAO, Y.** ; **HU, Z** ; **WU, X**. In-Vivo Induced CAR-T Cell for the Potential Breakthrough to Overcome the Barriers of Current CAR-T Cell Therapy. *Front. Oncol*, 2022, vol. 12, https://doi.org/10.3389/fonc.2022.809754